(19) Europäisches Patentamt
European Patent Office
Office européen des brevets

(11) Publication number: 0 043 139
B1

(12) EUROPEAN PATENT SPECIFICATION

(45) Date of publication of patent specification: 17.10.84

(21) Application number: 81105094.7

(22) Date of filing: 01.07.81

(51) Int. Cl.[3]: C 07 D 403/12, A 61 K 31/53 // (C07D403/12, 251/10, 241/20)

# (54) 3-Amino-5-substituted-6-halo-N-(3,4-dihydro- or 4,4-disubstituted-6-substituted-1,3,5-triazin-2-yl)-2-pyrazinecarboxamides, process for preparing and pharmaceutical compositions containing the same.

(30) Priority: 02.07.80 US 165312
02.07.80 US 165313

(43) Date of publication of application:
06.01.82 Bulletin 82/01

(45) Publication of the grant of the patent:
17.10.84 Bulletin 84/42

(84) Designated Contracting States:
AT BE CH DE FR GB IT LI LU NL SE

(56) References cited:
GB-A-1 242 625
US-A-3 647 794
US-A-3 972 882
US-A-4 098 892

(73) Proprietor: MERCK & CO. INC.
126, East Lincoln Avenue P.O. Box 2000
Rahway New Jersey 07065 (US)

(72) Inventor: Woltersdorf, Otto W., Jr.
200 Dorset Way
Chalfont Pennsylvania 18914 (US)
Inventor: Smith, Robert L.
1355 Pickwick Lane
Lansdale Pennsylvania 19446 (US)
Inventor: Cragoe, Edward J., Jr.
2211 Oak Terrace Drive
Lansdale Pennsylvania 19446 (US)
Inventor: De Solms, Jane S.
735 Port Indian Road
Norristown Pennsylvania 19403 (US)

(74) Representative: Abitz, Walter, Dr.-Ing. et al
Abitz, Morf, Gritschneder P.O. Box 86 01 09
D-8000 München 86 (DE)

EP 0 043 139 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

The background to this invention, U.S. Patent 3,313,813 patented April 11, 1967 and issued to Edward J. Craoge, Jr., shows novel (3-amino-5,6-disubstituted-pyrazinoyl)guanidine compounds. The compounds of the '813 patent are useful because they possess diuretic and natriuretic properties. They differ from most of the known, effective diuretic agents, however, in that the compounds of the '813 patent selectively enhance the excretion of sodium ions while simultaneously causing a decrease in excretion of potassium ions. The potassium loss, which is caused by known diuretics, often results in a severe muscular weakness. Since the compounds of the '813 patent prevent the potassium depletion, they have this decided advantage as diuretics. As diuretic agents, they can be used for the treatment of edema, hypertension and other diseases or conditions known to be responsive to this therapy and are especially useful when used in combination with or concomitantly with potassium losing diuretic agents.

It has been found in U.S. Patent 3,313,813 that the pyrazinoylguanidine compounds therein described when co-administered with other diuretic agents known to enhance the elimination of potassium ions along with sodium ions, will maintain the potassium ion excretion at approximately the normal or control rate and thus overcome this undesirable property of other diuretic agents.

In actuality, applicants' compounds in the instant case as further described, accomplish the objective previously achieved by using a combination of pyrazinoylguanidine compounds of the 3,313,813 patent with diuretic agents which cause elimination of sodium with concomitant excessive potassium elimination. Thus, the compounds of the instant case are effective eukalemic/saluretic agents. Since the compounds of the instant invention are thus eukalemic/saluretic agents, they constitute single entities which are useful for the treatment of edema and hypertension and other diseases or conditions known to be responsive to this therapy.

SUMMARY OF THE INVENTION

The instant case covers novel 3-amino-5-substituted-6-halo-N-(3,4-dihydro- or 4,4-disubstituted-6-substituted-1,3,5-triazin-2-yl)-2-pyrazinecarboxamides and processes for making the same. The novel compounds of this invention are depicted in Formula I below:

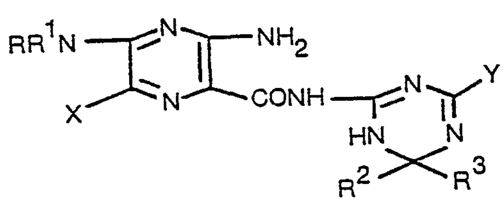

(I.)

wherein

R and $R^1$ are the same or different and are hydrogen or $C_{1-5}$ alkyl, $C_{2-5}$ alkenyl or $C_{3-6}$ cycloalkyl;

$R^2$ and $R^3$ are the same or different and are hydrogen or $C_{1-5}$ alkyl;

$R^2$ and $R^3$ may also be joined with the carbon atom to which they are attached to form a 3—7 membered carbon ring;

X is halo such as chloro, fluoro, bromo or iodo;

Y is OH, SH, $C_{1-5}$ alkoxy, $C_{1-5}$ alkylthio and $NR^4R^5$ wherein $R^4$ is hydrogen or $C_{1-5}$ alkyl and $R^5$ is hydrogen, $C_{1-5}$ alkyl, CN, $CONH_2$,

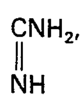, $NO_2$ or $NH_2$ .

and the pharmaceutically acceptable acid addition salts thereof.

In the above formula, $C_{1-5}$ alkyl means branched or straight chained alkyl groups such as methyl, ethyl, propyl, n-propyl, butyl, pentyl and isopentyl. $C_{1-5}$ alkenyl is represented by allyl, propenyl and the like and $C_{3-6}$ cycloalkyl is represented by cyclopropyl, cyclohexyl and cyclopentyl.

The invention includes compounds which are the tautomeric form of the compounds of Formula I namely to tautomeric forms of the formula:

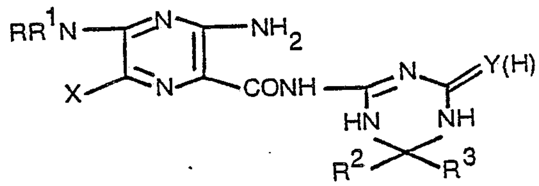

(II.)

wherein R, $R^1$, X, $R^2$, $R^3$, $R^4$ and $R^5$ are as defined for Formula I and Y is as defined above minus a hydrogen atom.

Preferred compounds of this invention are those compounds of Formula I and the tautomeric form of Formula II wherein

R and $R^1$ are hydrogen or $C_{1-5}$ alkyl;
$R^2$ and $R^3$ are methyl;
X is chloro; and
Y is OH, SH, $C_{1-5}$ alkoxy, $C_{1-5}$ alkylthio or cyanoamino

and the pharmaceutically acceptable salts thereof.

The compounds of this invention as shown by Formula I and the preferred compounds discussed above are useful because they possess diuretic and natriuretic properties. In addition, they are useful eukalemic saluretics, in other words, the compounds of the instant case cause neither loss or abnormal retention of potassium ions. In contradistinction, the pyrazinoylguanidine compounds of U.S. Patent 3,313,813 do cause a decrease in the excretion of potassium ions. However, other well known diuretics such as furosemide, chlorthalidone and acetazolamide cause an increase in potassium excretion which often results in muscular weakness. Applicants' compounds combine in a single agent the advantages of a combination of the known pyrazinoylguanidine diuretics of U.S. Patent 3,313,813 which decrease potassium loss with the known diuretics which cause a potassium loss. Thus, the compounds of this invention maintain the excretion of potassium at approximately normal levels while causing an increased renal elimination of sodium ions and water which is the desirable characteristic of the diuretic.

Also covered within the scope of the above Formula I compounds and the preferred compounds are the pharmaceutically acceptable acid addition salts thereof. These salts can be made by reacting the free base with a pharmaceutically acceptable acid such as for example, hydrochloric acid, sulfuric acid, hydrobromic acid or isethionic acid. These salts, as stated above, are to be considered as included in this invention.

The products of this invention can be administered to patients (both human and animal) in the form of pills, tablets, capsules, elixirs, injectable preparations and the like. They can be administered either orally or parentally or any other feasible method as known to those skilled in the art such as intravenously or in the form of suppositories.

The type of formulation to be administered can be comprised of one or more of the compounds of this invention as the only essential active ingredient of the pharmaceutical formulation. The formulation is merely combinations of the active ingredient mentioned with pharmaceutically inert carriers.

The compounds of this invention are advantageously administered at a dosage range of from 5 mg. to one gram per day or a somewhat higher or lower dosage at the physician's discretion, preferably in subdivided amounts on a 2 to 4 times a day regimen and most preferably at a dosage range from 10 to 500 mg. per day. It will be realized by those skilled in the art that the dosage range for any particular patient (animal or human) will depend upon the severity of the disease treated, weight of the patent and any other condition which the physician or other person skilled in the art will take account of.

The compounds of Formula I can be prepared by any of the following processes which are depicted by four chemical reaction schemes.

In all four reaction schemes R, $R^1$, $R^2$, $R^3$, $R^4$, $R^5$, X and Y are the same as is defined for Formulae I and II.

REACTION I

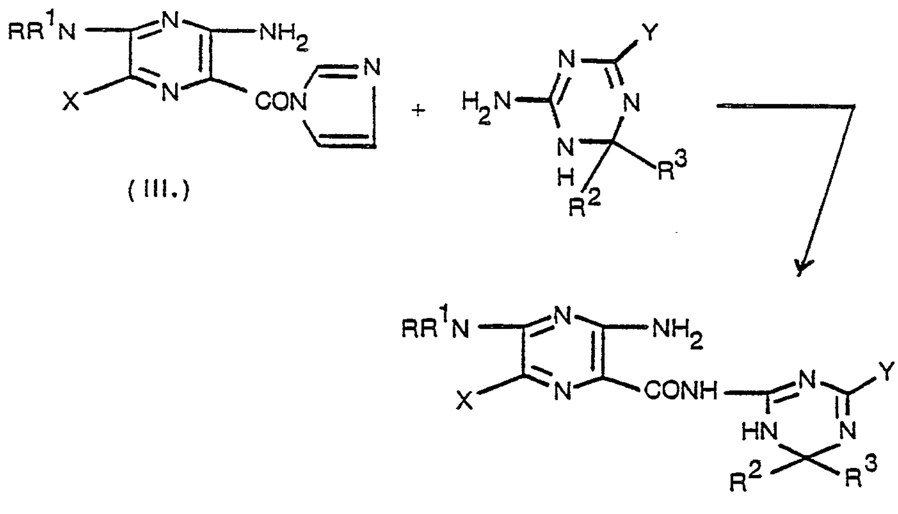

(III.)

(I.)

REACTION II

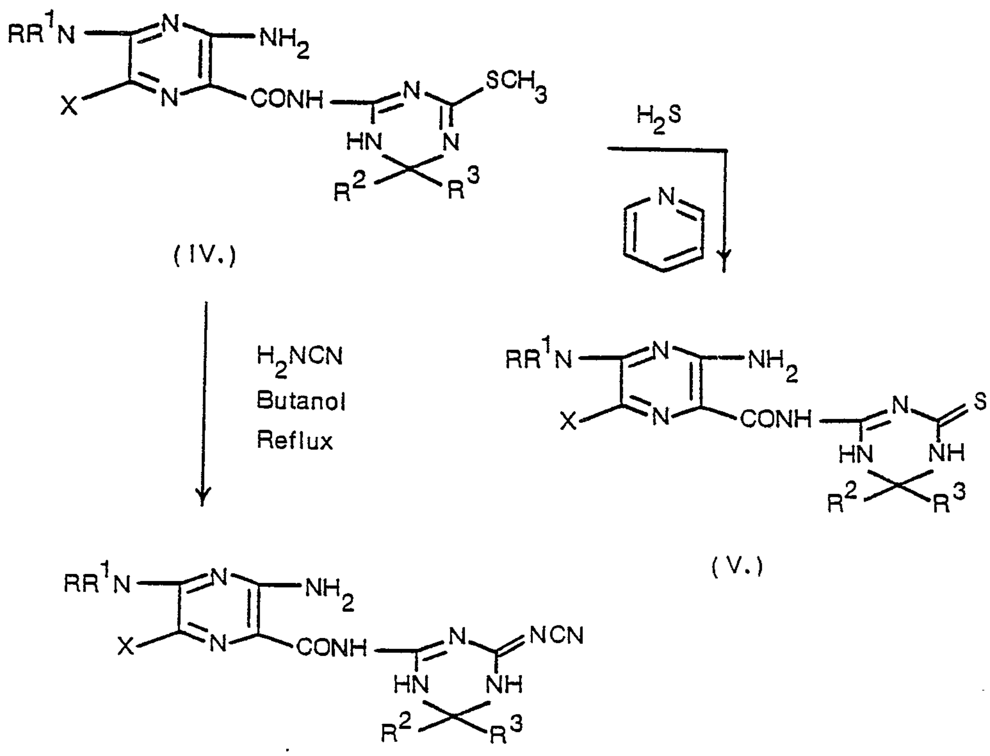


(IV.)

(V.)

(VI.)

REACTION III

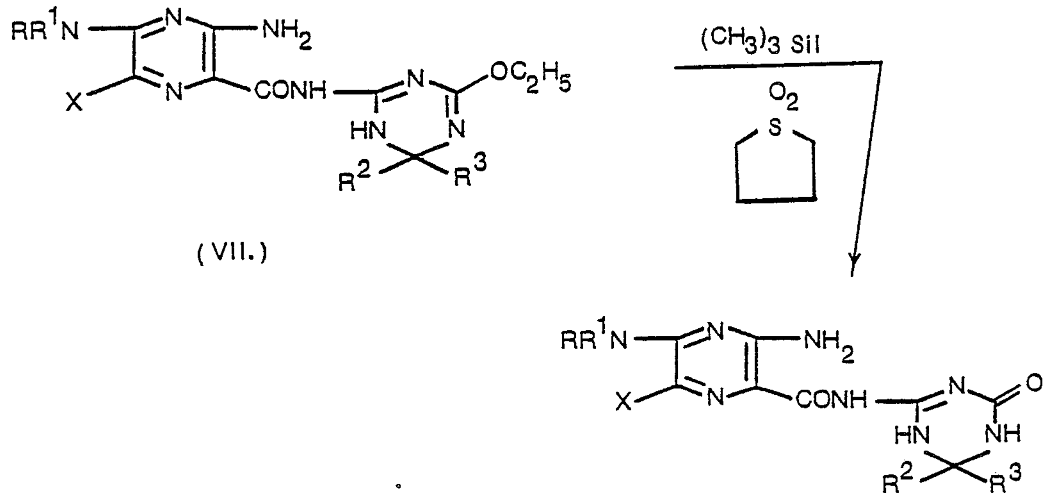


(VII.)

REACTION IV

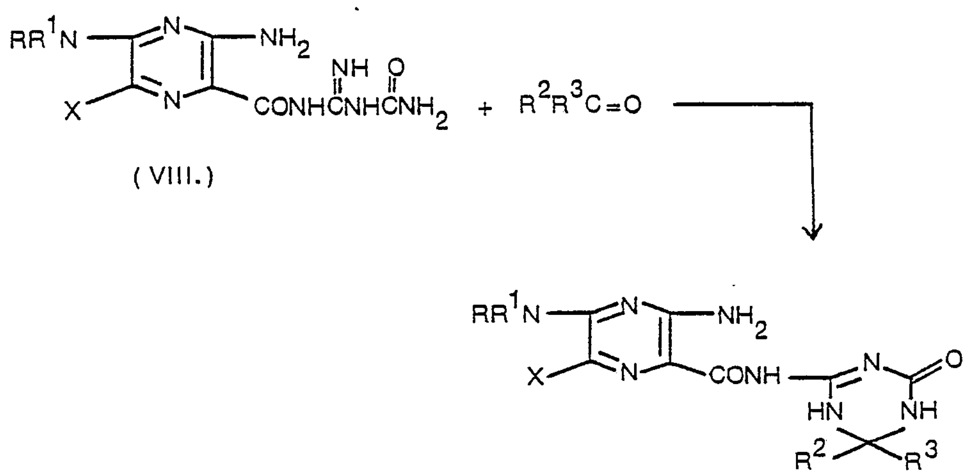

(VIII.)

(IX.)

The process in Reaction I involves reacting an active pyrazinoic acid amide such as the imidazole amide III with a 2-amino-3,4-dihydro-1,3,5-triazine to obtain the desired product. The reaction is conducted in a suitable solvent such as dimethylformamide, dimethylsulfoxide and the like at a temperature of from 25°C to the boiling temperature of the solvent but preferably from 50°C to 100°C. The reaction is usually conducted with an excess of the triazine component and the desired product is isolated by filtration of the cooled reaction or by treatment of the reaction solution with water followed by filtration of the product.

In Reaction II, the methylthio triazine carboxamide starting material IV is reacted with hydrogen sulfide gas in an organic solvent such as pyridine at a temperature of from 0—50°C for a period of 1—24 hours. The end product V is isolated from the reaction mixture by known means such as for example by treating the reaction mixture with water.

Similarly, the starting material IV can be dissolved in a solvent such as butanol and reacted with $H_2NCN$ to yield the product shown as Compound VI in Reaction Scheme II. This reaction is generally carried out at a temperature of 50° to reflux temperature of the solvent for a time of about 1—24 hours. The product VI is isolated by known means such as for example by cooling the reaction and filtering the product.

In Reaction III, the 6-ethoxy triazine carboxamide starting material VII is reacted with trimethyl silyl iodide in an organic solvent such as sulfolane at a temperature of from 25—100°C for a period of from 1—24 hours to yield 6-oxo triazine pyrazine carboxamide compounds.

Finally in Reaction IV starting material VIII is reacted with a ketone in a solvent such as dimethylformamide at 50° to reflux temperature of solvent for 1—24 hours. to yield a similar 6-oxo end product shown as Compound IX.

The following examples are included to illustrate the preparation of compounds of this invention and also to illustrate the preparation of a representative dosage form.

Example 1

3,5-Diamino-6-chloro-N-(3,4-dihydro-4,4-dimethyl-6-methylthio-1,3,5-triazin-2-yl)-2-pyrazinecarboxamide

To a solution of 3,5-diamino-6-chloropyrazinoic acid (1.13 g., 0.006 mole) in dimethylformamide (50 ml.) is added 1,1'carbonyldiimidazole (0.972 g., 0.006 mole). The reaction mixture is stirred for 1 hour in a nitrogen atmosphere to form, in situ, 1-(3,5-diamino-6-chloropyrazinoyl)imidazole, which is treated with 2-amino-3,4-dihydro-4,4-dimethyl-6-methylthio-1,3,5-triazine. The reaction mixture is heated at 95° for 3 hours and cooled to give 1.3 g. of 3,5-diamino-6-chloro-N-(3,4-dihydro-4,4-dimethyl-6-methylthio-1,3,5-triazin-2-yl)-2-pyrazinecarboxamide which melts at 232—4°C.

Elemental analysis for $C_{11}H_{15}ClN_8OS$:

| | |
|---|---|
| Calcd: | C, 38.54; H, 4.41; N, 32.69; |
| Found: | C, 38.39; H, 4.48; N, 32.64. |

Example 2

3,5-Diamino-6-chloro-N-(3,4-dihydro-4,4-dimethyl-6-mercapto-1,3,5-triazin-2-yl)-2-pyrazinecarboxamide hydrochloride

Hydrogen sulfide is a slowly bubbled with a solution of 3,5-diamino-6-chloro-N-(3,4-dihydro-4,4-dimethyl-6-methylthio-1,3,5-triazin-2-yl)-2-pyrazinecarboxamide (1.0 g., 0.0029 mole) in pyridine (25 ml.) containing triethylamine (1.5 ml.) for a period of five hours. Nitrogen is bubbled into the reaction mixture to remove excess hydrogen sulfide which is then poured into ice water (100 ml.). The collected solid is dissolved in hot water (75 ml.) containing hydrochloric acid (0.5 ml.) then cooled to give 3,5-diamino-6-chloro-N-(3,4-dihydro-4,4-dimethyl-6-mercapto-1,3,5-triazin-2-yl)-2-pyrazinecarboxamide hydrochloride which melts at 232°C with decomposition.

Elemental analysis for $C_{10}H_{13}ClN_8OS \cdot HCl$

| | |
|---|---|
| Calcd: | C, 32,88; H, 3.86; N, 30.68; |
| Found: | C, 32.93; H, 4.42; N, 30.94. |

Example 3

3,5-Diamino-6-chloro-N-(3,4-dihydro-4,4-dimethyl-6-cyanoamino-1,3,5-triazin-2-yl)-2-pyrazinecarboxamide

A solution of 3,5-diamino-6-chloro-N-(3,4-dihydro-4,4-dimethyl-6-methylthio-1,3,5-triazin-2-yl)-2-pyrazinecarboxamide (1.0 g., 0.0029 mol) and cyanamide (0.7 g., 0.017 mol) in 1-butanol (50 ml.) is heated at reflux for 5 hours. The 3,5-diamino-6-chloro-N-(3,4-dihydro-4,4-dimethyl-6-cyanoamino-1,3,5-triazin-2-yl)-2-pyrazinecarboxamide which separates melts at 342°C with decomposition.

Elemental analysis for $C_{11}H_{10}ClN_{10}O$;

| | |
|---|---|
| Calcd: | C, 39.23; H, 3.89; Cl, 10.53; |
| Found: | C, 39.32; H, 4.04; Cl, 10.65. |

Example 4

3,5-Diamino-6-chloro-N-(3,4-dihydro-4,4-dimethyl-6-ethoxy-1,3,5-triazin-2-yl)-2-pyrazinecarboxamide

*Step A:* 2-Amino-3,4-dihydro-4,4-dimethyl-6-ethoxy-1,3,5-triazine

To a solution of sodium (2.3g., 0.1 gr.atom) in absolute ethanol (100 ml.) is added 2-amino-3,4-dihydro-4,4-dimethyl-6-methylthio-1,3,5-triazine (3.4 g., 0.02 mole) and the mixture is heated at reflux for 18 hours. The ethanol is distilled at reduced pressure and the residue dissolved in water (100 ml.) and extracted with ethyl acetate (3 x 100 ml.) dried over potassium carbonate and evaporated *in vacuo* to give 1.3 g of 2-amino-3,4-dihydro-4,4-dimethyl-6-ethoxy-1,3,5-triazine which melts at 175—8°C after crystallization from acetonitrile.

Elemental analysis for $C_7H_{14}N_4O$:

| | |
|---|---|
| Calcd: | C, 49.39; H, 8.29; N, 32.92; |
| Found: | C, 48.84; H, 8.47; N, 33.42. |

*Step B:* The title compound is prepared by the process depicted in Reaction I.

Example 5

3,5-Diamino-6-chloro-N-(3,4-dihydro-4,4-dimethyl-6-hydroxy-1,3,5-triazin-2-yl)-2-pyrazinecarboxamide

To a stirred solution of 3,5-diamino-6-chloro-N-(3,4-dihydro-4,4-dimethyl-6-ethoxy-1,3,5-triazin-2-yl)-2-pyrazinecarboxamide (0.55 g., 0.0016 mole) in sulfone (10 ml.) is added trimethylsilyl iodide (1.5 ml.). The reaction mixture is stirred for 5 hours at 45°C, poured into ice water (30 ml.) containing hydrochloric acid (1.5 ml.), then extracted with chloroform (2 × 15 ml.) The aqueous layer is made basic with ammonia to give 420 mg. of 3,5-diamino-6-chloro-N-(3,4-dihydro-4,4-dimethyl-6-hydroxy-1,3,5-triazin-2-yl)-2-pyrazinecarboxamide which melts at 266—8°C. after reprecipitation from dilute hydrochloric acid with ammonia.

Elemental analysis for $C_{10}H_{13}ClN_8O_2$;

| | |
|---|---|
| Calcd. | C, 38.41; H, 4.19; N, 35.83; |
| Found: | C, 38.40, H, 4.25; N, 35.44. |

Example 6

3,5-Diamino-6-chloro-N-(3,4-dihydro-4,4-dimethyl-6-hydroxy-1,3,5-triazin-2-yl)-2-pyrazinecarboxamide

*Step A:* 2-Amino-3,4-dihydro-4,4-dimethyl-6-hydroxy-1,3,5-triazine

To a solution of 2-amino-3,4-dihydro-4,4-dimethyl-6-ethoxy-1,3,5-triazine (2.2 g.) in sulfolane (30 ml.) is added trimethylsilyl iodide (12 ml.). The reaction mixture is heated at 50°C for four hours then poured into 50 ml of ice water containing 2 ml. of conc. HCl. The solution is extracted with chloroform then the aqueous layer is distilled *in vacuo* to a viscous syrup which upon treatment with 2-propanol and cooling deposits 2.1 g. of product hydrochloride which is dissolved in 3 ml. of water and treated with 2 ml. of conc. ammonia to give 1.1 g. of 2-amino-3,4-dihydro-4,4-dimethyl-6-hydroxy-1,3,5-triazine which melts above 300°C.

Elemental Analysis for $C_5H_{10}N_4O$;

| | |
|---|---|
| Calcd: | C, 42.24; H, 7.09; N, 39.41; |
| Found: | C, 42.10; H, 7.51; N, 39.20. |

*Step B:* 3,5-Diamino-6-chloro-N-(3,4-dihydro-4,4-dimethyl-6-hydroxy-1,3,5-triazin-2-yl)-2-pyrazinecarboxamide

To a solution of 3,5-diamino-6-chloropyrazinoic acid (0.92 g., 0.0049 mole) in dimethylformamide (40 ml.) is added 1,1′-carbonyldiimidazole (0.80 g., 0.0049 mol). The reaction mixture is stirred for 2 hours in a nitrogen atmosphere to form, in situ, 1-(3,5-diamino-6-chloropyrazinoyl) imidazole which is treated with 2-amino-3,4-dihydro-4,4-dimethyl-6-hydroxy-1,3,5-triazine (1.1 g., 0.0076 mole). The reaction mixture is heated for 3 hours at 95°C, and treated with ice to give 1.3 g. of 3,5-diamino-6-chloro-N-(3,4-dihydro-4,4-dimethyl-6-hydroxy-1,3,5-triazin-2-yl)-2-pyrazinecarboxamide which melts at 266—8°C.

Elemental analysis for $C_{10}H_{13}ClN_8O_2$;

| | |
|---|---|
| Calcd: | C, 38.41; H, 4.19; N, 35.83; |
| Found: | C, 38.12; H, 4.51; N, 34.89. |

Example 7

Compressed Tablet containing 50 mg. of active ingredient.

| | Per Tablet, Mg. |
|---|---|
| 3,5-diamino-6-chloro-N-(3,4-dihydro-4,4-dimethyl-6-methylthio-1,3,5-triazin-2-yl)-2-pyrazinecarboxamide | 50 |
| Calcium phosphate dibasic | 200 |
| Ethyl cellulose (as 5% solution in ethanol) | 5 |
| Unmixed granulation | 255 |
| Add: | |
| Starch, corn | 14 |
| Magnesium stearate | 1 |
| | 270 |

*Directions:* Mix the active ingredient above and calcium phosphate and reduce to a No. 60 mesh powder. Granulate with Ethocel in alcohol and pass the wet granulation through a No. 10 screen (sieve opening 2.00 mm). Dry the granulation at 43.3°C for 12—18 hours. Dry grind to a No. 20 mesh (sieve opening 0.84.1 mm). Incorporate the "adds" and compress into tablets each weighing 270 mg.

Example 8

Dry filled capsule containing 50 mg. of active ingredient.

| | Per capsule, mg. |
|---|---|
| 3,5-diamino-6-chloro-N-(3,4-dihydro-4,4-dimethyl-6-mercapto 1,3,5-triazin-2-yl)-2-pyrazinecarboxamide hydrochloride | 50 |
| Lactose | 273 |
| Magnesium stearate | 2 |
| Mixed powders | 325 |

Mix the active ingredient above, lactose and magnesium stearate and reduce to a No. 60 mesh (sieve opening 0.250 mm) powder. Encapsulate, filling 325 mg. in each No. 2 capsule.

The above formulations can be employed to prepare compressed tablets or capsules of other novel compounds of this invention hereinbefore described.

Example 9

Combination dosage form in dry filled capsule

| | Per capsule, mg. |
|---|---|
| 3,5-diamino-6-chloro-N-(3,4-dihydro-4,4-dimethyl-6-cyanoamino-1,3,5-triazin-2-yl)-2-pyrazinecarboxamide | 50 |
| Magnesium stearate | 2 |
| lactose | 223 |
| Mixed powders | 275 |

Mix all of the above ingredients, reduce to a No. 60 mesh powder and encapsulate, filling 275 mg. in each No. 2 capsule.

**Claims for the Contracting States: BE CH DE FR GB IT LI LU NL SE**

1. A compound of the formula or the tautomeric form thereof

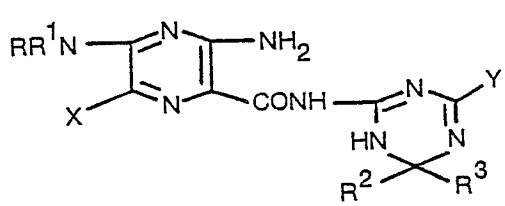

wherein

R is hydrogen, $C_{1-5}$ alkyl, $C_{2-5}$ alkenyl or $C_{3-6}$ cycloalkyl;

$R^1$ is hydrogen, $C_{1-5}$ alkyl, $C_{2-5}$ alkenyl or $C_{3-6}$ cycloalkyl;

$R^2$ is hydrogen, or $C_{1-5}$ alkyl;

$R^3$ is hydrogen, or $C_{1-5}$ alkyl;

$R^2$ and $R^3$ can be joined with the carbon atom to which they are attached to form a 3—7 membered carbon ring;

X is halo;

Y is OH, SH, $C_{1-5}$ alkoxy, $C_{1-5}$ alkylthio and $NR^4R^5$ wherein $R^4$ is hydrogen or $C_{1-5}$ alkyl and $R^5$ is hydrogen, $C_{1-5}$ alkyl, CN, $CONH_2$,

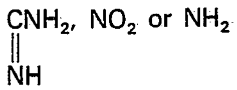

and the pharmaceutical acid addition salts thereof.

2. A compound of claim 1 having the formula

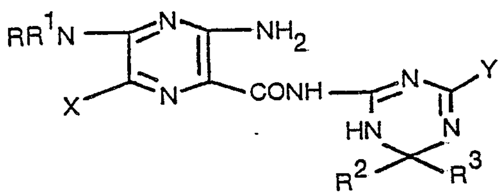

wherein

R is hydrogen or $C_{1-5}$ alkyl,
$R^1$ is hydrogen or $C_{1-5}$ alkyl,
$R^2$ is hydrogen or methyl,
$R^3$ is hydrogen or methyl,
X is chloro,
Y is OH, SH, $C_{1-5}$alkoxy, $C_{1-5}$ alkylthio or cyanoamino

and the pharmaceutically acceptable salts thereof.

3. A compound of claim 2 which is 3,5-diamino-6-chloro-N-(3,4-dihydro-4,4-dimethyl-6-methylthio-1,3,5-triazin-2-yl)-2-pyrazinecarboxamide.

4. A compound of claim 2 which is 3,5-diamino-6-chloro-N-(3,4-dihydro-6-hydroxy-1,3,5-triazin-2-yl)-pyrazinecarboxamide hydrate.

5. A pharmaceutical composition useful in the treatment of edema and hypertension which comprises in combination with a pharmaceutically acceptable carrier, a compound of claim 1.

6. A pharmaceutical composition according to claim 5 which comprises in combination with a pharmaceutically acceptable carrier, a compound of claim 2.

7. A process for preparing compounds of claim 1 which comprises reacting a compound of the formula:

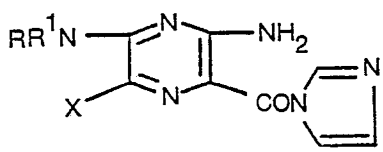

with a compound of the formula

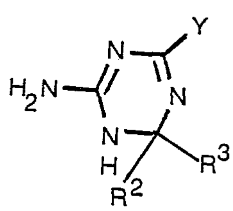

and, if desired, reacting the resulting compound with a pharmaceutically acceptable acid.

8. A process for preparing a compound of the formula

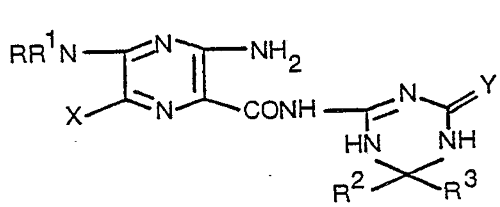

wherein R, $R^1$, $R^2$, $R^3$ and X are as defined in claim 1 and
Y is S or NCN
and the pharmaceutically acceptable acid addition salts thereof which comprises reacting a compound of the formula:

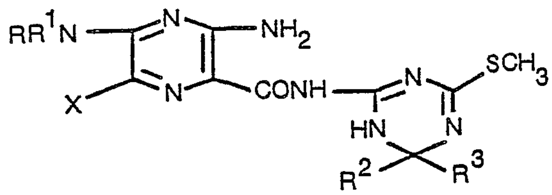

with $H_2S$ in pyridine to yield the desired compound when Y is S or with $H_2NCN$ to yield the desired product when Y is NCN, and, if desired, reacting the resulting compound with a pharmaceutically acceptable acid.

9. A process for preparing a compound of claim 1 having the formula

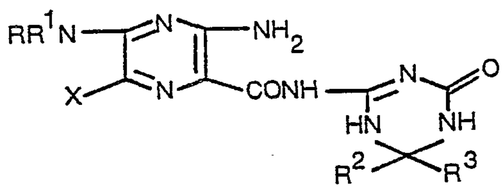

wherein R, $R^1$, $R^2$, $R^3$ and X are as defined in claim 1
and the pharmaceutically acceptable acid addition salts thereof which comprises reacting a compound of the formula

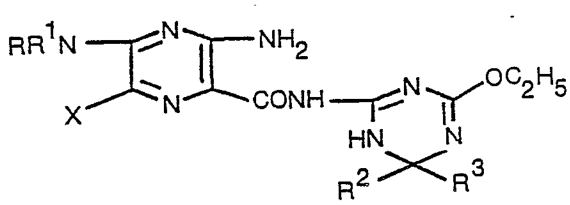

with $(CH_3)_3SiI$
to yield the desired product;
or by reacting a compound of the formula

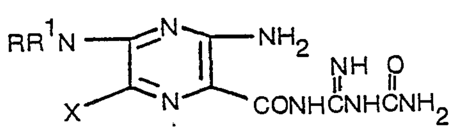

with $R^2R^3C = O$
to yield the desired product,
and, if desired, reacting the resulting compound with a pharmaceutically acceptable acid.

**Claims for the Contracting State: AT**

1. A process for preparing compounds of the formula:

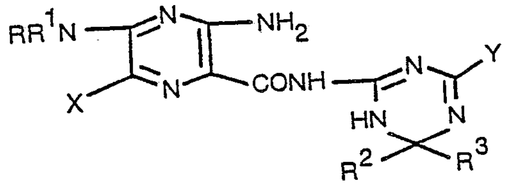

wherein

R is hydrogen, $C_{1-5}$ alkyl, $C_{2-5}$ alkenyl or $C_{3-6}$ cycloalkyl;

$R^1$ is hydrogen, $C_{1-5}$ alkyl, $C_{2-5}$ alkenyl or $C_{3-6}$ cycloalkyl;

$R^2$ is hydrogen, or $C_{1-5}$ alkyl;

$R^3$ is hydrogen, or $C_{1-5}$ alkyl;

$R^2$ and $R^3$ can be joined with the carbon atom to which they are attached to form a 3—7 membered carbon ring;

X is halo;

Y is OH, SH, $C_{1-5}$ alkoxy, $C_{1-5}$ alkylthio and $NR^4R^5$ wherein $R^4$ is hydrogen or $C_{1-5}$ alkyl and $R^5$ is hydrogen, $C_{1-5}$ alkyl, CN, $CONH_2$,

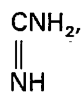, $NO_2$ or $NH_2$

and the pharmaceutically acceptable acid addition salts thereof which comprises reacting a compound of the formula:

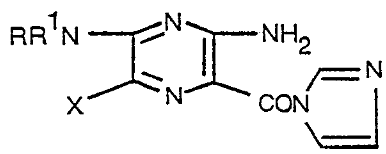

with a compound of the formula

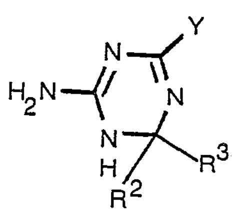

and, if desired, reacting the resulting compound with a pharmaceutically acceptable acid.

2. A process for preparing a compound of the formula

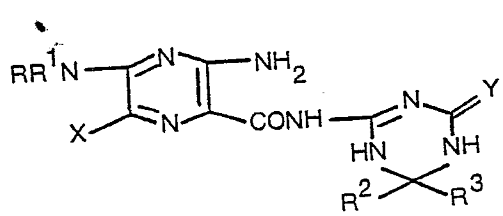

wherein R, $R^1$, $R^2$, $R^3$ and X are as defined in claim 1 and

Y is S or NCN

and the pharmaceutically acceptable acid addition salts thereof which comprises reacting a compound of the formula:

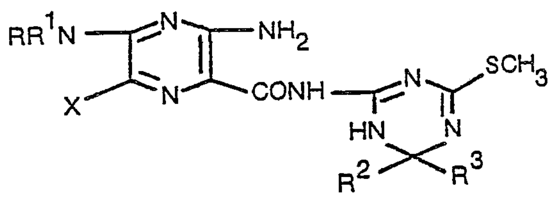

with $H_2S$ in pyridine to yield the desired compound when Y is S or with $H_2NCN$ to yield the desired product when Y is NCN, and, if desired, reacting the resulting compound with a pharmaceutically acceptable acid.

3. A process for preparing a compound of the formula

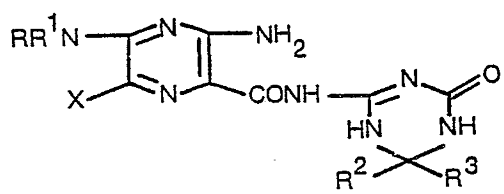

wherein R, $R^1$, $R^2$, $R^3$ and X are as defined in claim 1
and the pharmaceutically acceptable acid addition salts thereof which comprises reacting a compound of the formula

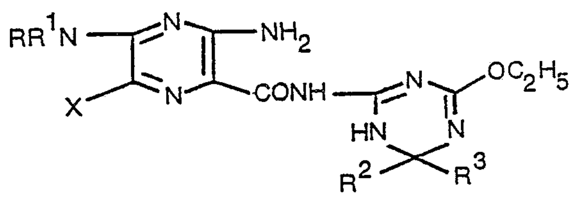

with $(CH_3)_3SiI$
to yield the desired product;
or by reacting a compound of the formula

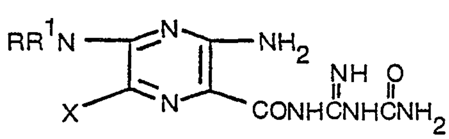

with $R^2R^3C = O$
to yield the desired product, and, if desired, reacting the resulting compound with a pharmaceutically acceptable acid.

**Patentansprüche für die Vertragsstaaten: BE CH DE FR GB IT LI LU NL SE**

1. Eine Verbindung der Formel

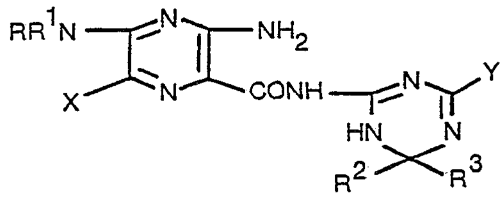

oder die tautomere Form davon, worin
R Wasserstoff, $C_{1-5}$-Alkyl, $C_{2-5}$-Alkenyl oder $C_{3-6}$-Cycloalkyl ist;
$R^1$ Wasserstoff, $C_{1-5}$-Alkyl, $C_{2-5}$-Alkenyl oder $C_{3-6}$-Cycloalkyl ist;
$R^2$ Wasserstoff oder $C_{1-5}$-Alkyl ist;
$R^3$ Wasserstoff oder $C_{1-5}$-Alkyl ist;
$R^2$ und $R^3$ mit dem Kohlenstoffatom, an das sie gebunden sind, unter Bildung eines 3- bis 7-gliedrigen Kohlenstoffringes verbunden sein können;
X Halogen ist;
Y OH, SH, $C_{1-5}$-Alkoxy, $C_{1-5}$-Alkylthio und $NR^4R^5$ ist, worin $R^4$ Wasserstoff oder $C_{1-5}$-Alkyl ist und $R^5$ Wasserstoff, $C_{1-5}$-Alkyl, CN, $CONH_2$,

$$\underset{\underset{NH}{\|}}{C}NH_2,\ NO_2 \text{ oder } NH_2 \text{ ist,}$$

und die pharmazeutischen Säureadditionssalze davon.

2. Eine Verbindung des Anspruchs 1 mit der Formel

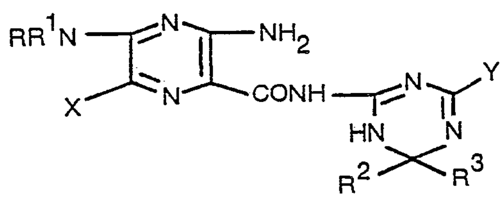

worin
R Wasserstoff oder $C_{1-5}$-Alkyl ist,
$R^1$ Wasserstoff oder $C_{1-5}$-Alkyl ist,
$R^2$ Wasserstoff oder Methyl ist,
$R^3$ Wasserstoff oder Methyl ist,
X Chlor ist,
Y OH, SH, $C_{1-5}$-Alkoxy, $C_{1-5}$-Alkylthio oder Cyanoamino ist,
und die pharmazeutische annehmbaren Salze davon.

3. Eine Verbindung des Anspruchs 2, welche 3,5-Diamino-6-chlor-N-(3,4-dihydro-4,4-dimethyl-6-methylthio-1,3,5-triazin-2-yl)-2-pyrazincarboxamid ist.

4. Eine Verbindung des Anspruchs 2, welche 3,5-Diamino-6-chlor-N-(3,4-dihydro-6-hydroxy-1,3,5-triazin-2-yl)-pyrazincarboxamid-hydrat ist.

5. Eine pharmazeutische Zusammensetzung, die für die Behandlung von Ödem und Hypertension brauchbar ist, umfassend eine Verbindung des Anspruchs 1 in Kombination mit einem pharmazeutisch annehmbaren Träger.

6. Eine pharmazeutische Zusammensetzung gemäß Anspruch 5, umfassend eine Verbindung des Anspruchs 2 in Kombination mit einem pharmazeutisch annehmbaren Träger.

7. Ein Verfahren zur Herstellung von Verbindungen des Anspruchs 1, umfassend die Umsetzung einer Verbindung der Formel:

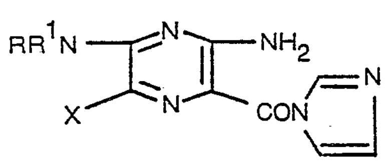

mit einer Verbindung der Formel

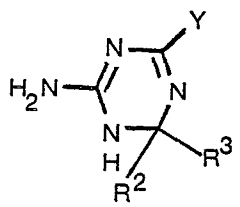

und gewünschtenfalls die Umsetzung der entstandenen Verbindung mit einer pharmazeutisch annehmbaren Säure.

8. Ein Verfahren zur Herstellung einer Verbindung der Formel

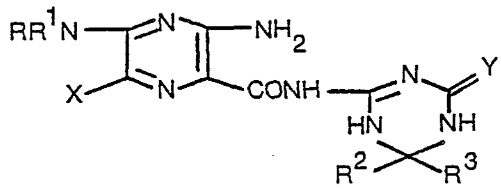

worin R, $R^1$, $R^2$, $R^3$ und X wie in Anspruch 1 definiert sind und Y S oder NCN ist, und der pharmazeutisch annehmbaren Säureadditionssalze davon, umfassend die Umsetzung einer Verbindung der Formel:

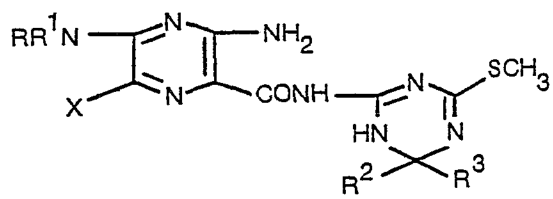

mit $H_2S$ in Pyridin unter Bildung der gewünschten Verbindung, falls Y S ist, oder mit $H_2NCN$ unter Bildung des gewünschten Produktes, falls Y NCN ist, und gewünschtenfalls Umsetzung der entstandenen Verbindung mit einer pharmazeutisch annehmbaren Säure.

9. Ein Verfahren zur Herstellung einer Verbindung des Anspruchs 1 mit der Formel

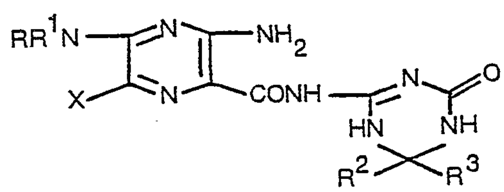

worin R, $R^1$, $R^2$, $R^3$ und X wie in Anspruch 1 definiert sind, und der pharmazeutisch annehmbaren Säureadditionssalze davon, umfassend die Umsetzung einer Verbindung der Formel

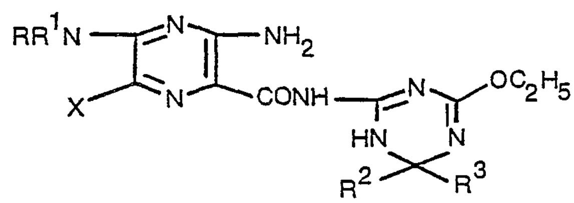

mit $(CH_3)_3SiI$
unter Bildung des gewünschten Produktes;
oder durch Umsetzung einer Verbindung der Formel

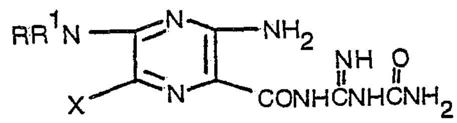

mit $R^2R^3C = O$ unter Bildung des gewünschten Produktes, und gewünschtenfalls die Umsetzung der entstandenen Verbindung mit einer pharmazeutisch annehmbaren Säure.

**Patentansprüche für den Vertragsstaat: AT**

1. Ein Verfahren zur Herstellung von Verbindung der Formel:

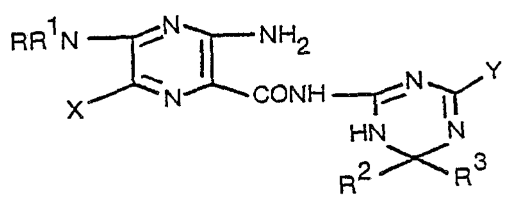

worin

R Wasserstoff, $C_{1-5}$-Alkyl, $C_{2-5}$-Alkenyl oder $C_{3-6}$-Cycloalkyl ist;
$R^1$ Wasserstoff, $C_{1-5}$-Alkyl, $C_{2-5}$-Alkenyl oder $C_{3-6}$-Cycloalkyl ist;
$R^2$ Wasserstoff oder $C_{1-5}$-Alkyl ist;
$R^3$ Wasserstoff oder $C_{1-5}$-Alkyl ist;
$R^2$ und $R^3$ mit dem Kohlenstoffatom, an das sie gebunden sind, unter Bilding eines 3- bis 7-gliedrigen Kohlenstoffringes verbunden sein können;
X Halogen ist;
Y OH, SH, $C_{1-5}$-Alkoxy, $C_{1-5}$-Alkylthio und $NR^4R^5$ ist, worin $R^4$ Wasserstoff oder $C_{1-5}$-Alkyl ist und $R^5$ Wasserstoff, $C_{1-5}$-Alkyl, CN, $CONH_2$,

$\underset{\underset{NH}{\parallel}}{C}NH_2$, $NO_2$ oder $NH_2$ ist,

und von pharmazeutisch annehmbaren Säureadditionssalzen davon, umfassend die Umsetzung einer Verbindung der Formel:

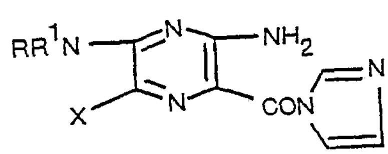

mit einer Verbindung der Formel

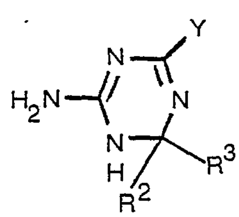

und gewünschtenfalls die Umsetzung der entstandenen Verbindung mit einer pharmazeutisch annehmbaren Säure.

2. Ein Verfahren zur Herstellung einer Verbindung der Formel

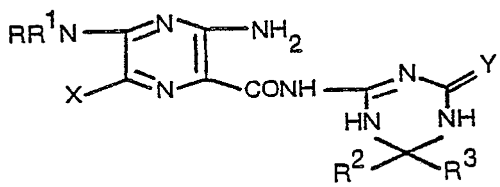

worin R, $R^1$, $R^2$, $R^3$ und X wie in Anspruch 1 definiert sind und Y S oder NCN ist, und der pharmazeutisch annehmbaren Säureadditionssalze davon, umfassend die Umsetzung einer Verbindung der Formel:

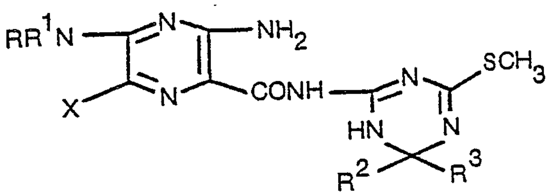

mit $H_2S$ in Pyridin unter Bildung der gewünschten Verbindung, falls Y S ist, oder mit $H_2NCN$ unter Bildung des gewünschten Produktes, falls Y NCN ist, und gewünschtenfalls Umsetzung der entstandenen Verbindung mit einer pharmazeutisch annehmbaren Säure.

3. Ein Verfahren zur Herstellung einer Verbindung der Formel

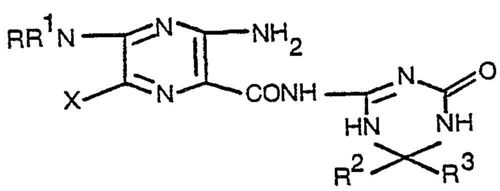

worin R, $R^1$, $R^2$, $R^3$ und X wie in Anspruch 1 definiert sind, und der pharmazeutisch annehmbaren Säureadditionssalze davon, umfassend die Umsetzung einer Verbindung der Formel

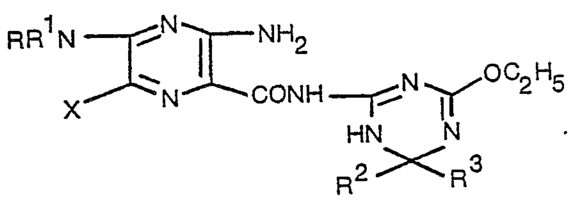

mit $(CH_3)_3SiI$
unter Bildung des gewünschten Produktes;
oder durch Umsetzung einer Verbindung der Formel

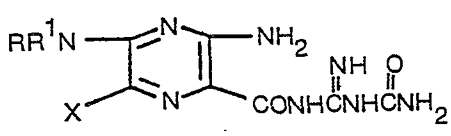

mit $R^2R^3C = O$
unter Bildung des gewünschten Produktes,
und gewünschtenfalls die Umsetzung der entstandenen Verbindung mit einer pharmazeutisch annehmbaren Säure.

**Revendications pour les Etats contractants: BE CH DE FR GB IT LI LU NL SE**

1. Composé de formule, ou la forme tautomère correspondante,

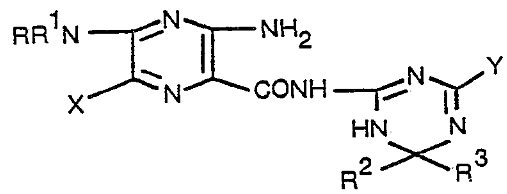

où:

R est un hydrogène, un alkyle en $C_{1-5}$, un alcényle en $C_{2-5}$ ou un cycloalkyle en $C_{3-6}$;
$R^1$ est un hydrogène, un alkyle en $C_{1-5}$, un alcényle en $C_{2-5}$, ou un cycloalkyle en $C_{3-6}$;
$R^2$ est un hydrogène ou un alkyle en $C_{1-5}$;
$R^3$ est un hydrogène ou un alkyle en $C_{1-5}$;
$R^2$ et $R^3$ peuvent être unis avec l'atome de carbone auquel ils sont fixés pour former un cycle carboné de 3 à 7 chaînons;
X est un halogéno;
Y est OH, SH, un alcoxy en $C_{1-5}$, un alkylthio en $C_{1-5}$ et $NR^4R^5$ où $R^4$ est un hydrogène ou un alkyle en $C_{1-5}$ et $R^5$ est un hydrogène, un alkyle en $C_{1-5}$, CN, $CONH_2$,

$CNH_2$, $NO_2$ ou $NH_2$
||
NH

et leurs sels d'addition d'acides acceptables en pharmacie.

2. Composé de la revendication 1 ayant pour formule:

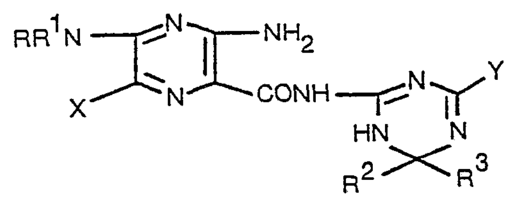

où:

R est un hydrogène ou un alkyle en $C_{1-5}$;
$R^1$ est un hydrogène ou un alkyle en $C_{1-5}$;
$R^2$ est un hydrogène ou un méthyle;
$R^3$ est un hydrogène ou un méthyle;
X est un chloro,
Y est OH, SH, un alcoxy en $C_{1-5}$, un alkylthio en $C_{1-5}$ ou un cyanoamino,
et leurs sels acceptables en pharmacie.

3. Composé de la revendication 2 qui est le 3,5-diamino-6-chloro-N-(3,4-dihydro-4,4-diméthyl-6-méthylthio-1,3,5-triazin-2-yl)-2-pyrazine-carboxamide.

4. Composé de la revendication 2 qui est le 3,5-diamino-6-chloro-N-(3,4-dihydro-6-hydroxy-1,3,5-triazin-2-yl)-2-pyrazine carboxamide hydraté.

5. Composition pharmaceutique utile dans le traitement de l'oedème et de l'hypertension qui comprend en combinaison avec un support acceptable en pharmacie un composé de la revendication 1.

6. Composition pharmaceutique selon la revendication 5 qui comprend une combinaison avec un support acceptable en pharmacie d'un composé de la revendication 2.

7. Procédé pour préparer les composés de la revendication 1 qui comprend la réaction d'un composé de formule:

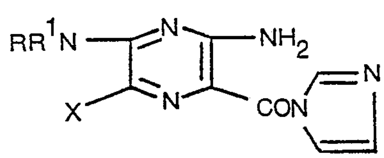

avec un composé de formule:

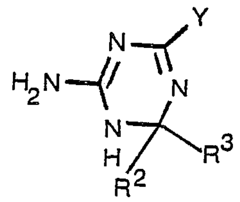

et, si on le désire, la réaction du composé obtenu avec un acide acceptable en pharmacie.

8. Procédé pour préparer un composé de formule:

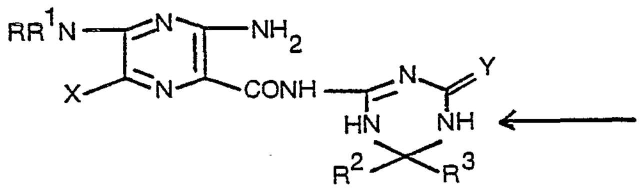

dans laquelle:

$R$, $R^1$, $R^2$, $R^3$ et X sont comme définis dans la revendication 1, et

Y est S ou NCN,

et leurs sels d'addition d'acides acceptables en pharmacie qui comprend la réaction d'un composé de formule:

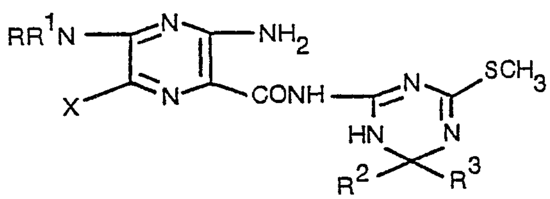

avec $H_2S$ dans la pyridine pour obtenir le composé désiré lorsque Y est S ou avec $H_2NCN$ pour obtenir le composé désiré lorsque Y est NCN, et, si on le désire, la réaction du composé obtenu avec un acide acceptable en pharmacie.

9. Procédé pour préparer un composé de la revendication 1 ayant pour formule:

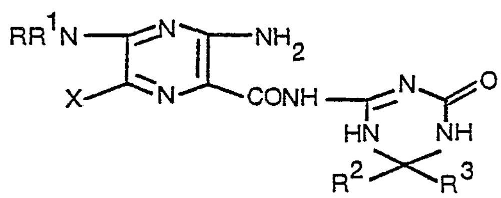

dans laquelle: $R$, $R^1$, $R^2$, $R^3$ et X sont comme définis dans la revendication 1,
et leurs sels d'addition d'acides acceptables en pharmacie qui comprend la réaction d'un composé de formule:

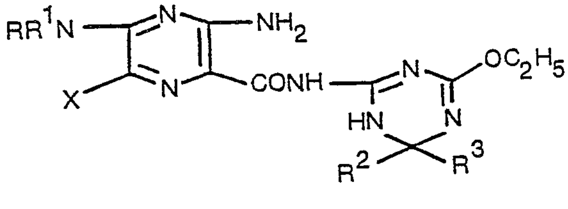

avec $(CH_3)_3SiI$
pour fournir le produit désiré;

ou la réaction d'un composé de formule:

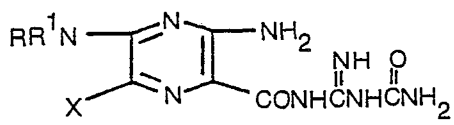

avec $R^2R^3C = O$
pour fournir le produit désiré;
et, si on le désire, le réaction du composé obtenu avec un acide acceptable en pharmacie.

**Revendications pour l'Etat contractant: AT**

1. Procédé pour préparer les composés de formule:

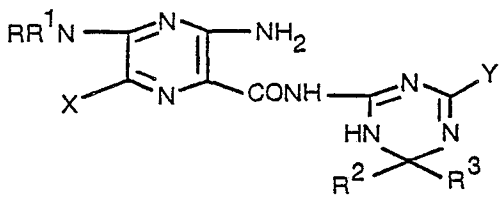

où:

R est un hydrogène, un alkyle en $C_{1-5}$, un alcényle en $C_{2-5}$ ou un cycloalkyle en $C_{3-6}$;
$R^1$ est un hydrogène, un alkyle en $C_{1-5}$, un alcényle en $C_{2-5}$ ou un cycloalkyle en $C_{3-6}$;
$R^2$ est un hydrogène ou un alkyle en $C_{1-5}$;
$R^3$ est un hydrogène ou un alkyle en $C_{1-5}$;
$R^2$ et $R^3$ peuvent être unis avec l'atome de carbone auquel ils sont fixés pour former un cycle carboné de 3 à 7 chaînons;
X est un halogéno;
Y est OH, SH, un alcoxy en $C_{1-5}$, un alkylthio en $C_{1-5}$ et $NR^4R^5$ où $R^4$ est un hydrogéne ou un alkyle en $C_{1-5}$ et $R^5$ est un hydrogéne, un alkyle en $C_{1-5}$, CN, $CONH_2$,

$CNH_2$, $NO_2$ ou $NH_2$,
||
NH

et leurs sels d'addition d'acides acceptables en pharmacie, qui comprend la réaction d'un composé de formule:

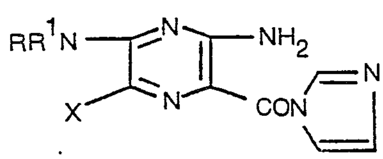

avec un composé de formule:

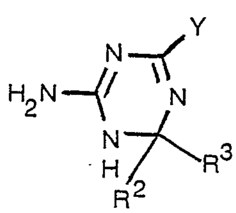

et, si on le désire, la réaction du composé obtenu avec un acide acceptable en pharmacie.

2. Procédé pour préparer un composé de formule:

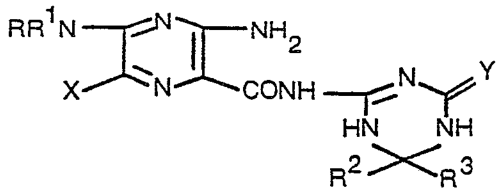

dans laquelle:

R, $R^1$, $R^2$, $R^3$ et X sont comme définis dans la revendication 1, et

Y est S ou NCN,

et leurs sels d'addition d'acides acceptables en pharmacie, qui comprend la réaction d'un composé de formule:

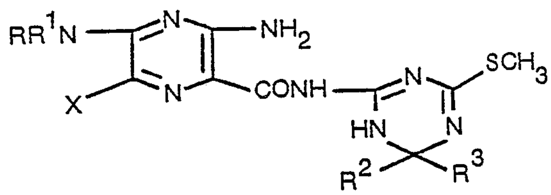

avec $H_2S$ dans la pyridine pour fournir le composé désiré lorsque Y est S ou avec $H_2NCN$ pour fournir le produit désiré lorsque Y est NCN, et, si on le désire, la réaction du composé obtenu avec un acide acceptable en pharmacie.

3. Procédé pour préparer un composé de formule:

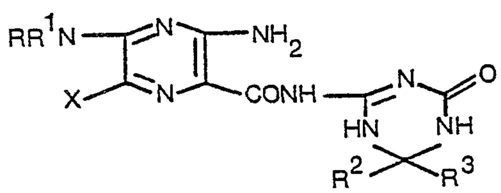

dans laquelle: R, $R^1$, $R^2$, $R^3$ et X sont comme définis dans la revendication 1,
et leurs sels d'addition d'acides acceptables en pharmacie, qui comprend la réaction d'un composé de formule:

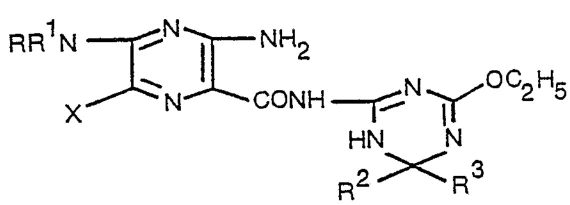

avec $(CH_3)_3SiI$

pour fournir le produit désiré;

ou la réaction d'un composé de formule:

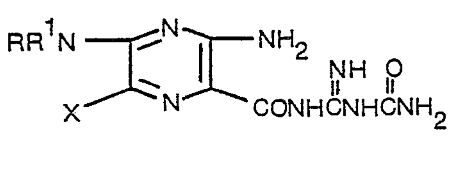

avec $R^2R^3C = O$

pour fournir le produit désiré;

et, si on le désire, la réaction du composé obtenu avec un acide acceptable en pharmacie.